# EUROPÄISCHE PATENTSCHRIFT

(11) **EP 3 251 718 B1**
(45) Veröffentlichungstag und Bekanntmachung des Hinweises auf die Patenterteilung: **15.08.2018**
(21) Anmeldenummer: 16172474.5
(22) Anmeldetag: 01.06.2016
(51) Int. Cl.: A61M 16/08, A61M 39/10

(54) **ATEMWEGSTHERAPIEGERÄT**
AIRWAY THERAPY APPARATUS
APPAREIL DE SOINS RESPIRATOIRES

(43) Veröffentlichungstag der Anmeldung: 06.12.2017
(73) Patentinhaber: R. Cegla GmbH & Co. KG, 56410 Montabaur (DE)
(72) Erfinder: Cegla, Ulrich, 56410 Montabaur (DE); Ebinger, Andrea, 56410 Montabaur (DE)
(74) Vertreter: Engelhardt, Volker

(56) Entgegenhaltungen:
- WO-A1-98/24500
- US-A- 4 557 261
- US-A1- 2009 199 853
- US-A1- 2013 160 888
- US-A1- 2014 238 389
- US-A1- 2016 045 689

## Beschreibung

Die Erfindung bezieht sich auf ein Atemwegstherapiegerät nach dem Oberbegriff des Patentanspruches 1.

Ein derartiges Atemwegstherapiegerät ist der EP 2 087 927 A1 zu entnehmen. Das Atemwegstherapiegerät dient zur Verbesserung der Atmung eines Menschen und besteht typischerweise aus einem Mundstück und einem Schlauch, der auf einer Tülle auf dem Mundstück aufgezogen ist. Darüber hinaus ist der Schlauch von einem gebogenen bzw. gekrümmten Rohrstück umgeben, das die Krümmung des Schlauches vorgibt. Beim Ausatmen ist die Atemluft in den Schlauch eingepresst und in Abhängigkeit von der Krümmung des Rohrstückes ist dieser zu einer Schwingung angeregt.

Derartige Atemwegstherapiegeräte, die beispielsweise aus der US 2013/160888 A1, der US 2014/238389 A1 oder der WO 98/24500 A1 bekannt geworden sind, haben sich in der Praxis vielseitig bewährt und werden zur Therapie von Asthma-Patienten mit erheblichen Atemwegsproblemen bis hin zum Spitzensportler zur Vergrößerung des Lungenvolumens und zur Verbesserung des Atmungsvorganges verwendet.

Nachteiligerweise hat sich jedoch herausgestellt, dass der Austausch des Schlauches ein gewisses Maß an Fingerfertigkeit voraussetzt, da der Schlauch aus einem elastischen Kunststoff auf die Tülle des Rohrstückes bzw. des Mundstückes aufgezogen werden muss. Hierzu muss der Schlauch zunächst aufgedehnt werden um in Anschluss den Schlauch auf die Tülle zu stülpen. Insbesondere stellt der Austausch des Schlauches für Patienten mit eingeschränkter Fingermobilität oder einer geringen Fingerfertigkeit ein wesentliches Hindernis dar.

Der Erfindung liegt daher die Aufgabe zugrunde, ein Atemwegstherapiegerät zur Behandlung von Atemwegserkrankungen der vorgenannten Gattung zur Verfügung zu stellen, dessen Schlauch einfach und unkompliziert, auch für Personen mit eingeschränkter Fingermobilität oder Fingerfertigkeit, austauschbar ist, mit dessen Hilfe der Patient die Atemwege beim Ein- bzw. Ausatmen trainieren kann und dadurch der gewünschte Therapieeffekt eintritt.

Darüber hinaus ist es Aufgabe der Erfindung, ein Atemwegstherapiegerät bereit zu stellen, dessen einzelne Komponenten kostengünstig, einfach zu handhaben und benutzerfreundlich zu reinigen bzw. zu sterilisieren sind.

Diese Aufgaben werden durch die Merkmale des kennzeichnenden Teils von Patentanspruch 1 gelöst.

Weitere vorteilhafte Weiterbildungen der Erfindung ergeben sich aus den Unteransprüchen.

Dadurch dass der Umfang des ersten freien Endes des Schlauches im Verhältnis zu dem Umfang des Durchlasskanals von dem freien Ende des Rohrstücks kleiner oder gleich bemessen ist, dass die Breite einer der Längsseiten des ersten freien Endes des Schlauches größer bemessen ist als die Breite bzw. der Durchmesser des Durchlasskanals von dem freien Ende des Rohrstücks, dass der Schlauch durch eine Stauchung der Längsseite in den Durchlasskanals bereichsweise einsteckbar ist und dass der Schlauch im eingesetzten Zustand durch eine Vorspannkraft in dem Durchlasskanal gehalten ist, kann der Schlauch einfach und unkompliziert in den Durchlasskanal des Rohrstückes auch von Personen mit eingeschränkter Fingermobilität oder Fingerfertigkeit eingesetzt werden. Der Schlauch kann manuell ohne ein Aufdehnen durch ein geringfügiges Zusammendrücken bzw. Stauchen der Längsseiten in das Rohrstück eingesetzt werden. Nach dem Einsetzten und dem Loslassen streb der Schlauch aufgrund seiner elastischen Eigenschaften in die Ausgangsform zurück und die Längsseiten werden durch den Spannungszustand gegen den Durchlasskanal gedrückt. Der Schlauch legt sich an den Durchlasskanal an, so dass die ausgeatmete Atem-Luft in den Schlauch geleitet ist und diesen beim Durchströmen zum Schwingen bringt.

Darüber hinaus hat es sich als vorteilhaft erwiesen, dass an dem ersten freien Ende des Schlauches, auf der dem Durchlasskanal zugewandten Seite, eine oder mehrere Anformungen angeordnet ist bzw. sind. Die Anformungen stehen nach Außen von dem Schlauch ab und dichten in Form einer Lamellendichtung im eingesetzten Zustand den Luftspalt zwischen dem Schlauch und dem Durchlasskanal. Ein Durchströmen der Atemluft durch den Luftspalt zwischen dem Schlauch und dem Rohrstück ist somit weitestgehend verhindert.

In dem Durchlasskanal können vorteilhafterweise im Bereich des ersten freien Endes des Schlauches ein oder mehrere Nuten oder Absätze eingearbeitet bzw. angearbeitet sein, die ebenfalls eine Lamellendichtung zwischen dem Schlauch und dem Rohrstück bilden. Ferner können die Nuten an die Abmessungen der Anformungen an dem Schlauch angepasst sein, so dass die Anformungen des Schlauches in die Nuten greifen. Der Schlauch ist somit einerseits durch den Formschluss in dem Durchlasskanal gehalten und zum anderen ist durch die Anordnung eine gute Dichtwirkung der Lamellendichtung erreicht.

Weiterhin hat es sich als vorteilhaft erwiesen, den Durchlasskanal durch mindestens eine Durchlasskanalabzweigung in mehrere Durchlasskanalzweige zu teilen. In jeden der Durchlasskanalzweige ist ein Ventil angeordnet, das die Strömungsrichtung in dem jeweiligen Durchlasskanalzweig vorgibt. In jedem der Durchlasskanalzweige kann somit in Abstimmung mit der Strömungsrichtung ein Schlauch eingesetzt sein, so dass die Durchlasskanalzweige wechselseitig durch die Ventile beim Ein- bzw. Ausatmen durchströmt sind und der jeweilige Schlauch schwingt.

Der Schlauch ist vorteilhafterweise als Flach-Schlauch oder oval-förmiger Schlauch ausgebildet. Der Durchlasskanal ist dafür rotationsymmetrisch, wobei die Breite des Flachschlauches größer bemessen ist als der Durchmesser des Durchlasskanals.

Das erste freie Ende des Schlauchs kann auch oval -förmig sein, wobei der Umfang des Schlauches keiner oder gleich dem Umfang des Durchlasskanals ist und die breiteste Längsseite des Schlauches größer bemessen ist als der Durchmesser des Schlauches oder vice versa.

In der Zeichnung sind ein erfindungsgemäßes Ausführungsbeispiel und zwei weitere Ausführungsvarianten dargestellt, die nachfolgend näher erläutert sind. Im Einzelnen zeigt:
- Figur 1a: ein Atemwegstherapiegerät mit einem Rohrstück, das einen Durchlasskanal bildet, an dessen ersten freien Ende ein Mundstück angearbeitet bzw. angeformt ist und ein Schlauch in den Durchlasskanal des zweiten freien Endes eingesetzt ist,
- Figur 1b: das Atemwegstherapiegerät gemäß Figur 1a, wobei Atemluft in Strömungsrichtung durch das Mundstück eingeblasen ist und der Schlauch zum Schwingen angeregt ist,
- Figur 2a und 2b: Drauf- und Seitenansicht des Schlauches gemäß Figur 1a,
- Figur 3: eine Schnittdarstellung quer zur Strömungsrichtung gemäß Figur 1a,
- Figur 4: eine Schnittdarstellung quer zur Strömungsrichtung gemäß Figur 1b,
- Figur 5: eine Schnittdarstellung des Atemwegstherapiegerätes gemäß Figur 1a,
- Figur 6: Schnittdarstellung einer zweiten Ausführungsvariante gemäß Figur 1a mit in den Durchlasskanal eingearbeiteten Nuten, in die von dem Schlauch abstehende Anformungen eingreifen und
- Figur 7: eine dritte Ausführungsvariante des erfindungsgemäßen Atemwegstherapiegerätes mit einer Durchlasskanalabzweigung und zwei Ventilen, die jeweils beim Ein- bzw. Ausblasen und vice versa den jeweiligen Kanalzweig durchströmen lassen.

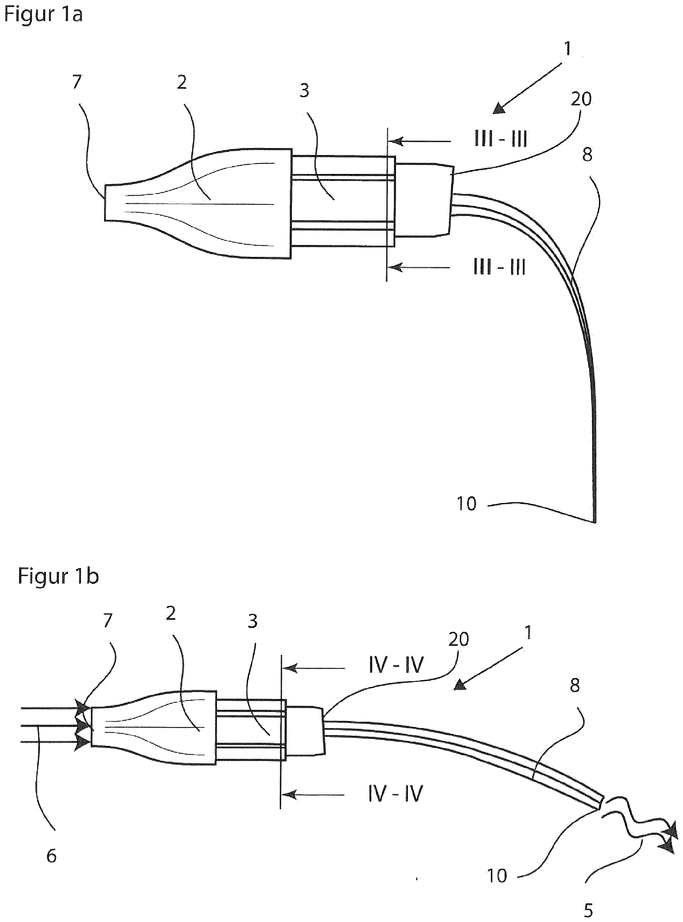

In Figur 1a ist ein Atemwegstherapiegerät 1 zur Verbesserung der Atmung eines Patienten abgebildet, das aus einem Rohrstück 3 gebildet ist. Das Rohrstück 3 weist im Inneren einen zylinderförmigen Durchlasskanal 4 mit einen Durchmesser d auf. An dem ersten freien Ende des Rohrstückes 3 ist ein Mundstück 2 angebracht, welches einerseits für den Patienten eine angenehme orale Aufnahme des Atemwegstherapiegerätes 1 schafft und andererseits eine Öffnung für den Durchlasskanal 4 bildet. Auf der dem Mundstück 2 abgewandten Seite des Rohrstückes 3 ist ein Schlauch 8 in den Durchlasskanal 4 teilweise eingeführt bzw. eingesetzt.

Der Figur 1b ist das Atemwegstherapiegerät 1 im betätigten Zustand zu entnehmen. Durch die Öffnung 7 des Mundstückes 2 ist in eine Strömungsrichtung 6 Atemluft in den Durchlasskanal 4 eingepresst. Die Atem-Luft 5 durchströmt den Durchlasskanal 4 und gelangt durch diesen in den Schlauch 8. Die Luft 5 weitet bei einer ausreichenden Luftströmung den Schlauch 8 auf und versetzt diesen in eine oszillierende Schwingung.

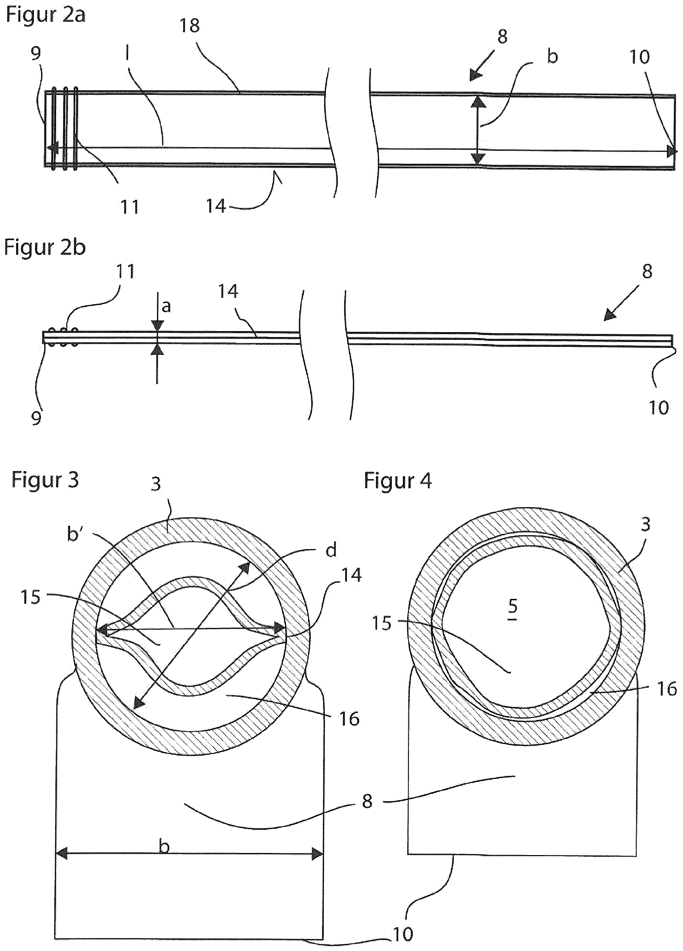

Damit der Schlauch 8 auch für Patienten mit eingeschränkter Fingermobilität unkompliziert mit dem Durchlasskanal 4 zusammenführbar ist und bei Benutzung nicht ungewollt herausrutscht, ist der Schlauch 8 durch eine kraft- und/oder formschlüssige Verbindung bzw. eine Vorspannkraft in dem Durchlasskanal 4, wie nachfolgend durch die Figuren 2a bis 5 dargestellt, gehalten. Nach dem Gebrauch des Atemwegstherapiegerät 1 kann der Schlauch 8 zu Reinigungszwecken oder zur Entsorgung aus dem Durchlasskanal 4 mit nur geringen Kraftaufwand herausgezogen werden.

Der Schlauch 8 hierzu ist, und das ist insbesondere den Figuren 2a und 2b zu entnehmen, als ein Flach-Schlauch 8 oder Oval-Schlauch (b>>a) ausgeformt, an dessen ersten freien Ende 9 mehrere Anformungen 11 angeformt oder angearbeitet sind. Entlang der beiden Längsseiten 14 verbindet eine Schlauchnaht 18 jeweils die untere und obere Hälfte des Flach-Schlauches 8 miteinander. Die Innenseiten des Schlauches 8 sind mit einem Trennmittel versehen, dass ein Zusammenkleben der beiden Innenseiten des Schlauches 8 verhindert.

Der Schlauch 8 ist aus einem elastischen Material, vorzugweise Silikon, hergestellt. Die Breite b - der Abstand zwischen den beiden Längsseiten 14 - des Schlauches 8 ist größer bemessen als der Durchmesser d des Durchlasskanals 4 und die Länge I des Schlauches 8 entspricht dem Vielfachen seiner Breite b.

Der Flach-Schlauch 8 wird zum Einführen in den Durchflusskanal 4 an dem ersten freien Ende 9 zusammengedrückt, so dass die Breite b' des Flach-Schlauches 8 nunmehr kleiner ist als der Durchmesser d des Durchlasskanals 4 (b>d>b'). Der Schlauch 8 wird anschließend in den Durchlasskanal 4 bereichsweise eingesteckt bzw. eingeführt und sobald dieser losgelassen ist, strebt der Schlauch 8 aufgrund seiner elastischen Eigenschaften in die ursprüngliche Form zurück. Da jedoch der Durchmesser d des Durchlasskanals 4 kleiner bemessen ist als die Breite b des Schlauches 8 im Ausgangszustand, werden die Längsseiten 14 des Schlauches 8 gegen die beiden sich gegenüberliegenden Seiten des Durchlasskanals 4 gepresst, wodurch die kraftschlüssige bzw. reibschlüssige Verbindung zwischen dem Rohrstück 3 und dem Schlauch 8, so wie in der Figur 3 dargestellt, zustande kommt. Die beiden Längsseiten 14 des ersten freien Endes 9 des Schlauches 8 liegen somit im eingesteckten Zustand bereichsweise an dem Durchlasskanal 4 an.

Der Schlauch 8 muss hierfür aus mindestens einem elastischen Werkstoff hergestellt sein und ein ausreichende Steifigkeit I aufweisen. Die die Vorspannkraft, die die kraftschlüssige Verbindung zwischen dem Schlauch 8 und dem Durchlasskanal 4 bildet, muss ausreichend groß bemessen, um den Schlauch 8 auch bei hohen Durchströmungsgeschwindigkeiten bzw. maximalen Atemluft-Druck in dem Durchlasskanal 4 zu halten.

Typischer Weise ist das erste freie Ende des Schlauches 8 zwischen 0,25d bis 2d tief in den Durchlasskanal 4 eingesteckt, jedoch kann diese Maßangabe in Abhängigkeit von zahlreichen Faktoren stark variieren. Wesentliche Parameter sind die Vorspannkraft als eine Funktion von der Elastizität und Steifigkeit (EI) des Schlauches 8, der Einstecktiefe und den Reibung zwischen Schlauch 8 und Durchlasskanal 4.

Durch das Zusammendrücken der Längsseiten 14 vor dem Einsetzen des Flach-Schlauches 8 in den Durchlasskanal 4 ist die obere und untere Seite an dem ersten freien Ende 9 voneinander getrennt und es bildet sich eine Einlassöffnung 15 im Schlauch 8. Beim Ausatmen, und dies ist insbesondere der Figur 4 zu entnehmen, ist der Schlauch 8 vollständig durch die Durchströmung geöffnet und gegen den Durchlasskanal 4 gedrückt.

Aufgrund der Ausgestaltung des Schlauches 8 ist nach wie vor ein geringfügiger Luftspalt 16 zwischen dem Schlauch 8 und den Durchlasskanal 4 vorhanden, da der Umfang des Schlauches 8 kleiner bemessen ist, als der Umfang des Durchlasskanals 4. Um diesen Luftspalt 16 bestmöglich zu dichten, sind auf der dem Durchlasskanal 4 zugewandten Seite des Schlauches 8 mehrere Anformungen 11 angeformt, die nach Art einer Lamellendichtung 13 den Luftspalt 16 bestmöglich dichten.

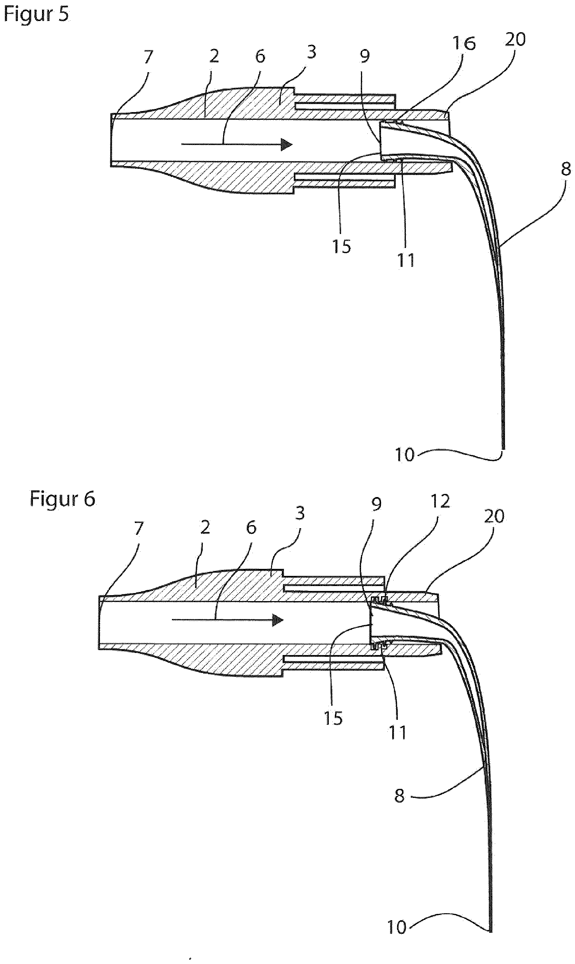

Insbesondere ist der Figur 5 zu entnehmen, dass der Schlauch 8 mindestens so tief in den Durchlasskanal 4 eingeschoben werden muss bis das alle Anformungen 11 an dem Durchlasskanal 4 anliegen. Die Einsetztiefe zusätzlich kann durch Markierungen auf dem Schlauch 8 angezeigt werden.

Der Figur 6 ist eine weitere Ausführungsvariante des Atemwegstherapiegerätes 1 zu entnehmen, wobei in dem Durchlasskanal 4 mehrere Nuten 12 eingearbeitet sind. Die Nuten 12 bilden einerseits die Lamellendichtung 13 des Luftspaltes 16 zwischen dem Durchlasskanal 4 und dem Schlauch 8. Andererseits können die an die Abmessungen der Anformungen 11 des Schlauches 8 angepasst sein, so dass die Anformungen 11 im eingesetzten Zustand des Schlauches 8 in die Nuten 12 eingreifen. Somit ist zusätzlich zu dem Kraftschluss ein Formschluss gebildet.

Der Schlauch wird folglich einerseits durch die Vorspannkraft, die die beiden Längsseiten 14 gegen die beiden sich gegenüberliegenden Seiten des Durchlasskanals gepresst, gehalten und anderseits durch den Formschluss zwischen den Anformungen 11 und den Nuten 12.

Die Nuten 12 können auch ohne weiteres als Absätze 17 ausgebildet sein, die aus dem Rohrstück 3 in den Durchlasskanal 4 ragen. Die Absätze 17 können einerseits einen Anschlag für den Schlauch 8 bilden und/oder andererseits die Lamellendichtung 13 formen.

Alternativ kann der Durchlasskanal 4 des Rohrstückes 3 konisch ausgebildet sein, dessen Durchmesser d am zweiten freien Ende kontinuierlich aufgeweitet ist, so dass der Schlauch 8 in diesem beim Einführen verklemmt werden kann.

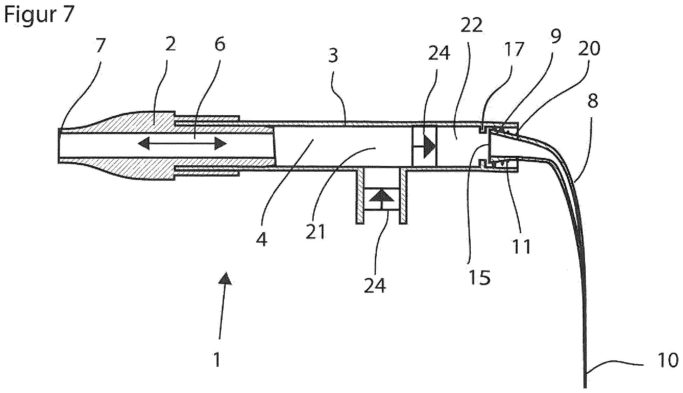

Der Figur 7 ist eine zweite Ausführungsvariante des erfindungsgemäßen Atemwegstherapiegerätes 1 zu entnehmen. Das Atemwegstherapiegerät 1 ist aus einer Vielzahl von Rohrstücken 3 gebildet die modular zusammengesteckt sind. Die Rohrstücke 3 weisen jeweils eine Steckeinrichtung 25 auf die es ermöglicht, zwei Rohrstücke 3 luftdicht miteinander zu verbinden.

In einem der dem Rohrstück 3 eine Durchlasskanal-Abzweigung 21 angeordnet ist, die den Durchlasskanal 4 in einen ersten und einen zweiten Durchlasskanal-Zweig 22, 23 aufteilt. Das Rohrstück 3 ist mittels der Steckeinrichtung 25 auf das Mundstück 2 aufgesetzt und weist zwei Ventile 24 auf, die dem jeweiligen Durchlasskanal-Zweig 22, 23 eine Strömungsrichtung 6 vorgeben. Demnach ist beim Ausatmen durch die Ventile 24 der erste Durchlasskanal-Zweig 22 freigegeben und die Atem-Luft 5 ist durch den ersten Durchlasskanal-Zweig 22 zu dem Schlauch 8 geleitet. Beim Einatmen schließt das Ventil 24 in dem ersten Durchlasskanal-Zweig 22 und das Ventil 24 in dem zweiten Durchlasskanal-Zweig 23 ist geöffnet, so dass der Patient mit dem Atemwegstherapiegerät 1 sowohl aus- als auch einatmen kann.

In dem zweiten Durchlasskanal-Zweig 23 kann ohne weiteres ein zweiter Schlauch 8 angeordnet sein, dessen zweites freies Ende 10 im Durchlasskanal 4 in Richtung des Mundstückes 2 gerichtet ist.

Das erste freie Ende 9 des Schlauches 8 kann konisch ausgebildet sein, so dass die Einlasssöffnung 15 des Schlauches 8 geringfügig geöffnet ist und eine geöffnete Einlassöffnung 15 aufweist. Der Schlauch 8 bzw. dessen erstes freies Ende 9 ist im Querschnitt somit oval (a<b), wobei die Breite b des Schlauches 8 größer ist als der Durchmesser d des Durchlasskanals 4 (b>d) und der Umfang des Schlauches 8 keiner oder gleich dem Umfang des Durchlasskanals 4 ist (2·b ≤ Π·d).

Der Durchlasskanal 4 kann auch nicht rotationssymmetrische Querschnittformen z.B. rechteckig, dreieckig oder dergleichen aufweisen. Ohne Weiteres sind insbesondere ovale Querschnitte möglich deren Verhältnis Breite:Höhe (a:b) auf das Öffnungsverhältnis des Mundes des Patienten angepasst sein kann.

Wesentlich ist, dass der Umfang des ersten freien Endes 9 des Schlauches 8 kleiner dimensioniert ist als der Innenumfang des Rohrstückes 3 bzw. als der Umfang des Durchlasskanals 8 und die Breite b einer der Längsseiten 14 des Schlauches 8 größer bemessen ist als der Abstand der möglichen Kontaktpunkte der Längsseiten 14 des Schlauches 8 in dem Durchlasskanal 4. Die Kontaktpunkte sind auf zwei gegenüberliegenden Seiten des Durchlasskanals angeordnet, wobei eine Verbindungslinie der beiden Punkte den Flächenmittelpunkt des Durchlasskanals schneidet.

## Patentansprüche

1. Atemwegstherapiegerät (1) zur Behandlung von Atemwegserkrankungen bestehend aus:
- mindestens einem Rohstücken (3), das mindestens einen Durchlasskanal (4) aufweist, durch den Luft (5) ein- bzw. ausatembar ist
- mindestens einem elastischen Schlauch (8), dessen erstes freies Ende (9) an einem freien Ende an einem der Rohrstücke (4) angeordnet ist und beim Ein- bzw. Ausatmen in eine Strömungsrichtung (6) durch eine Durchströmung schwingen kann
**dadurch gekennzeichnet,**
**dass** der Umfang des ersten freien Endes (9) des Schlauches (8) im Verhältnis zu dem Umfang des Durchlasskanals (4) von dem freien Ende des Rohrstücks (3) kleiner oder gleich groß bemessen ist, dass die Breite (b) einer der Längsseiten (14) des ersten freien Endes (9) des Schlauches (8) größer bemessen ist als die Breite bzw. der Durchmesser (d) des Durchlasskanals (4), dass der Schlauch (8) durch Stauchung der Längsseite (14) in den Durchlasskanals (4) bereichsweise einsteckbar ist und dass der Schlauch (8) im eingesetzten Zustand durch eine Vorspannkraft in dem Durchlasskanal (4) gehalten ist.

2. Atemwegstherapiegerät (1) nach Anspruch 1
**dadurch gekennzeichnet,**
**dass** an dem ersten freien Ende (9) des Schlauchs (8) auf der dem Durchlasskanal (4) zugewandten Seite ein oder mehrere Anformungen (11) angeordnet sind und dass die Anformungen (11) in dem Durchlasskanal (4) eine lamellendichtung (12) bilden.

3. Atemwegstherapiegerät (1) nach Anspruch 2
**dadurch gekennzeichnet,**
**dass** in dem Durchlasskanal (4) im Bereich des ersten freien Endes (9) des Schlauches (8) ein oder mehrere Nuten (12) eingearbeitet sind und dass die Nuten (12) in dem Durchlasskanal (4) die Lamellendichtung (13) zwischen dem Schlauch (8) und dem Rohrstück (3) bilden.

4. Atemwegstherapiegerät (1) nach einem der Ansprüche 2 oder 3,
**dadurch gekennzeichnet,**
**dass** die Nut (12) an die Abmessungen der Anformung (11) an dem Schlauch (8) angepasst ist, dass die Anformung (11) in die Nut (12) eingreift und dass der Schlauch (8) durch den Formschluss zwischen der Anformung (11) und der Nut (12) in dem Durchlasskanal (4) gehalten ist.

5. Atemwegstherapiegerät (1) nach einem der vorgenannten Ansprüche
**dadurch gekennzeichnet,**
**dass** das erste freien Ende (9) des Schlauchs (8) eine Trichterform aufweist und dass durch die Trichterform eine Einlassöffnung (15) gebildet ist.

6. Atemwegstherapiegerät (1) nach einem der vorgenannten Ansprüche
**dadurch gekennzeichnet,**
**dass** der Durchlasskanal (4) durch mindestens eine Durchlasskanal-Abzweigung (21) in mehrere Durchlasskanalzweige (22, 23) unterteilt ist, dass in jedem der Durchlasskanalzweig (22, 23) ein Ventil (24) angeordnet ist und dass durch das Ventil (24) die Strömungsrichtung (5) in dem jeweiligen Rohrstücken (3) vorgegeben ist.

7. Atemwegstherapiegerät (1) nach Anspruch 6
**dadurch gekennzeichnet,**
**dass** in jeden der Durchlasskanalzweige (22, 23) ein Schlauch (8) eingesetzt ist und dass zwei der Durchlasskanalzweige (22, 23) wechselseitig durch das Ventil (24) beim Ein- bzw. Ausatmen durchströmt sind und der jeweilige Schlauch (8) schwingt..

8. Atemwegstherapiegerät (1) nach einem der vorgenannten Ansprüche
**dadurch gekennzeichnet,**
**dass** der Schlauch (8) als flach- oder oval förmiger Schlauch (8) geformt ist..

9. Atemwegstherapiegerät (1) nach einem der vorgenannten Ansprüche
**dadurch gekennzeichnet,**
**dass** der Schlauch (8) aus Silikon hergestellt ist.

10. Atemwegstherapiegerät (1) nach einem der vorgenannten Ansprüche
**dadurch gekennzeichnet,**
**dass** an einem der Rohrstücke (3) ein Mundstück (2) angeordnet ist.

## Claims

1. An airway therapy apparatus (1) for the treatment of respiratory problems, comprising:
- at least one pipe section (3) which has at least one passage duct (4) through which air (5) can be inhaled or exhaled
- at least one elastic hose (8) with its first free end (9) arranged on one free end on one of the pipe sections (3) and which can vibrate during inhalation or exhalation in one flow direction (6) because of the throughflow,
**characterised in that,**
the circumference of the first free end (9) of the hose (8) is smaller than or equal to the circumference of the passage duct (4) of the free end of the pipe section (3), that the width (b) of one of the long sides (14) of the first free end (9) of the hose (8) is larger than the width or diameter (d) of the passage duct (4), that areas of the hose (8) can be inserted into the passage duct (4) by compressing the long side (14) and that the hose (8) is held in the passage duct (4) by a preload force after having been inserted.

2. The airway therapy apparatus (1) in accordance with Claim 1,
**characterised in that,**
one or more bulges (11) are arranged on the first free end (9) of the hose (8) on the side facing the passage duct (4) and that the bulges (11) form a lamellar seal (13) in the passage duct (4).

3. The airway therapy apparatus (1) in accordance with Claim 2,
**characterised in that,**
one or more grooves (12) are worked into the passage duct (4) in the area of the first free end (9) of the hose (8) and that the grooves (12) in the passage duct (4) form the lamellar seal (13) between the hose (8) and the pipe section (3).

4. The airway therapy apparatus (1) in accordance with one of Claims 2 or 3,
**characterised in that,**
the groove (12) is adapted to the dimensions of the bulge (11) on the hose (8), that the bulge (11) engages in the groove (12) and that the hose (8) is held in the passage duct (4) by the form-locked connection between the bulge (11) and the groove (12).

5. The airway therapy apparatus (1) in accordance with one of the aforementioned claims,
**characterised in that,**
the first free end (9) of the hose (8) has a funnel shape and that an inlet opening (15) is formed by the funnel shape.

6. The airway therapy apparatus (1) in accordance with one of the aforementioned claims,
**characterised in that,**
the passage duct (4) is subdivided into several passage duct branches (22, 23) by at least one passage duct branching (21), that a valve (24) is arranged in each of the passage duct branches (22, 23) and that the valve (24) establishes the flow direction (5) in the particular pipe sections (3).

7. The airway therapy apparatus (1) in accordance with Claim 6,
**characterised in that,**
a hose (8) is inserted in each of the passage duct branches (22, 23) and that air flows alternately within two of the passage duct branches (22, 23) during inhalation and exhalation because of the valve (24), causing the hose (8) in question to vibrate.

8. The airway therapy apparatus (1) in accordance with one of the aforementioned claims,
**characterised in that,**
the hose (8) is formed as a flat or oval-shaped hose (8).

9. The airway therapy apparatus (1) in accordance with one of the aforementioned claims,
**characterised in that,**
the hose (8) is manufactured from silicone.

10. The airway therapy apparatus (1) in accordance with one of the aforementioned claims,
**characterised in that,**
a mouthpiece (2) is arranged on one of the pipe sections (3).

## Revendications

1. Appareil thérapeutique (1) servant au traitement de maladies des voies respiratoires, comprenant :
- au moins un bout de tuyau (3) dans lequel il est pratiqué au moins un canal de passage (4) permettant d'aspirer ou d'expirer de l'air (5),
- au moins un tuyau flexible (8) dont la première extrémité libre (9) est montée sur une extrémité libre d'un des bouts de tuyau (3) et qui peut osciller à la respiration ou à l'expiration lors du passage du flux (6) dans une direction,
**caractérisé en ce que**
le pourtour de la première extrémité libre (9) du tuyau flexible (8) est plus petit que ou égal au pourtour du canal de passage (4) de l'extrémité libre du bout de tuyau (3), que la largeur (b) d'une des côtés longitudinaux (14) de la première extrémité libre (9) du tuyau flexible (8) est plus grand que la largeur ou le diamètre (d) du canal de passage (4), qu'en refoulant le côté longitudinal (14), le tuyau flexible (8) se laisse insérer partiellement dans le canal de passage (4), et qu'en état assemblé, le tuyau flexible (8) est retenu par le tarage dans la canal de passage (4).

2. Appareil thérapeutique pour voies respiratoires (1) d'après la revendication 1,
**caractérisé en ce que**
sur le côté de la première extrémité libre (9) du tuyau flexible (8) donnant sur le canal de passage (4), il est prévu un ou plusieurs embouts (11) et que les embouts (11) forment un étoupage à lamelles (13) dans le canal de passage (4).

3. Appareil thérapeutique pour voies respiratoires (1) d'après la revendication 2,
**caractérisé en ce que**,
au niveau de la première extrémité libre (9) du tuyau flexible (8), il est pratiqué une ou plusieurs gorges (12) dans le canal de passage (4) et que les gorges (12) dans le canal de passage (4) forment l'étoupage à lamelles (13) entre le tuyau flexible (8) et le bout de tuyau (3).

4. Appareil thérapeutique pour voies respiratoires (1) d'après une des revendications 2 ou 3,
**caractérisé en ce que**
la gorge (12) est adaptée aux dimensions de l'embout (11) sur le tuyau flexible (8), que l'embout (11) s'engrène dans la gorge (12) et que grâce à la connexion par la forme entre l'embout (11) et la gorge (12), le tuyau flexible (8) est retenu dans le canal de passage (4).

5. Appareil thérapeutique pour voies respiratoires (1) d'après une des revendications précédentes,
**caractérisé en ce que**
la première extrémité libre (9) du tuyau flexible (8) a la forme d'une trémie et que cette trémie constitue une ouverture d'entrée (15).

6. Appareil thérapeutique pour voies respiratoires (1) d'après une des revendications précédentes,
**caractérisé en ce que**,
moyennant au moins une ramification du canal de passage (21), le canal de passage (4) est subdivisé un plusieurs branches de canal de passage (22, 23), que chacune des branches du canal de passage (22, 23) contient une soupape (24) et que cette soupape (24) détermine le sens du flux (5) dans le bout de tuyau respectifs (3).

7. Appareil thérapeutique pour voies respiratoires (1) d'après la revendication 6,
**caractérisé en ce que**
dans chacune des branches du canal de passage (22, 23), il est monté un tuyau flexible (8) et que, lors de la respiration et l'expiration, la soupape (24) assure le passage alternatif dans les deux des canaux de passage (22, 23) et le tuyau flexible respectif (8) oscille.

8. Appareil thérapeutique pour voies respiratoires (1) d'après une des revendications précédentes,
**caractérisé en ce que**
le tuyau flexible (8) est conçu sous la forme d'un tuyau flexible (8) plat ou ovale.

9. Appareil thérapeutique pour voies respiratoires (1) d'après une des revendications précédentes,
**caractérisé en ce que**
le tuyau flexible (8) est fabriqué en silicone.

10. Appareil thérapeutique pour voies respiratoires (1) d'après une des revendications précédentes,
**caractérisé en ce que**
sur un des bouts de tuyau (3), il est prévu un bec (2).
